# EUROPEAN PATENT APPLICATION

(11) **EP 3 928 838 A1**
(43) Date of publication of application: **29.12.2021**
(21) Application number: 20181412.6
(22) Date of filing: 22.06.2020
(51) Int. Cl.: A61Q 19/00, A61K 8/891, A61K 8/892

(54) **FOOT OIL**

(71) Applicant: Magnifeet B.V., 1731 RD Winkel (NL)
(72) Inventor: Regenboog-den Hollander, Eveline Charlotte, 1731 RD Winkel (NL)
(74) Representative: Octrooibureau Los en Stigter B.V.

(57) **Abstract**

The invention is directed to a foot oil consisting of 75.0 - 99.9 wt% dimethicone, 25.0 - 0.1 wt% dimethiconol and optionally 1.10⁻⁶ - 0.1 wt% additives based on the total amount of foot oil and to the use of the foot oil for protection of the skin against friction, wherein the friction can be caused by footwear.

## Description

The invention is directed to a foot oil.

The feet are under heavy load all day. Not only the joints and muscles in the feet, but also the skin of the feet needs extra attention and care. Blisters, inflammations, warm and irritated feet are often the cause of a stressed skin by wearing shoes. Further, foot and toe problems, like bunions (hallux valgus), hammer toes and mallet toes cause pain when walking, running or standing for a longer time.

New bought shoes are often tight and it is difficult to put on the shoes. The shoes do not fit well and this is caused by the fact that the feet cannot move properly in the shoes and will swell. Plasters and socks are not sufficient for a long term protection of the skin of the feet and protect the feet only temporarily and/or topical.

There are many foot gels and creams that keep the skin of the foot in good condition and protect it against dryness and cracking. However, these gels and creams do not protect the skin of the feet against friction.

The foot oil according to the present invention is surprisingly effective for the protection of the skin of the feet against friction. By using the foot oil according to the invention blisters, skin irritation and swelling of the foot will be strongly reduced or will not occur. Also, the pain caused by foot and toe problems will decrease.

The foot oil according to the invention consists of 75.0 - 99.9 wt% dimethicone, 25.0 - 0.1 wt% dimethiconol and optionally 1.10⁻⁶ - 0.1 wt% additives based on the total amount of foot oil.

Dimethicone and dimethiconol are silicone polymers and are known for use in cosmetic products, such as hair care products or skin care products.

Dimethicone is also known as polydimethylsiloxane (PDMS) or dimethylpolysiloxane. Dimethicone and dimethiconol are normally used in low concentrations up to 15 wt% in cosmetic products.

An example of a cosmetic foot product comprising polydimethylsiloxane and dimethiconol is described in CN106176264. The foot product described herein comprises only 2 wt% of polydimetylsiloxane and 3 wt% of dimethiconol. Dimethicone is a polymeric silicone with chemical structure A:

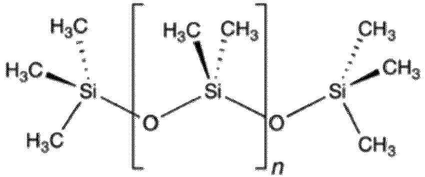

Dimethiconol is similar to dimethicone in its chemical structure, except that molecules of dimethiconol end with hydroxy (-OH) groups. This means that in the end groups of chemical structure A one of the -CH3 groups is replaced by an -OH group.

In structure A the repeating unit can occur n times. This means that dimethicone and/or dimethiconol in the foot oil can be one silicone type with a fixed amount of repeating units. However, dimethicone and/or dimethiconol can also be present as a mixture of different silicone polymers with varying amounts of n repeating units. These silicone polymers will have different lengths and also different properties.

In the foot oil according to the invention the amount of dimethicone is 75.0 - 99.9 wt%, preferably 80-99 wt% and more preferably 85-97 wt% based on the total amount of foot oil.

The amount of dimethiconol in the foot oil according to the invention is 25.0 - 0.1 wt%, preferably 20-1.0 wt% and more preferably 15-3.0 wt% based on the total amount of foot oil.

The optional amount of additives in the foot oil according to the invention is1.10⁻⁶ - 0.1 wt% based on the total amount of foot oil. Additives can, for example, be fragrances, perfumes, aromatic substances, colorants, dyes, pigments, stabilizers, preservatives and antioxidants; like vitamin C and E.

The foot oil preferably has a density of 0.920 to 0.940 g/cm³, more preferably a density of 0.922 to 0.938 g/cm³.

The invention is also directed to the preparation of the foot oil. The foot oil is prepared by mixing at least one dimethicone, at least one dimethiconol and optionally the additives to form the foot oil. Preferably, at least one dimethicone has a density of 0.895 to 0.925 g/cm³, more preferably all dimethicones have a density of 0.895 to 0.925 g/cm³.

The invention if further directed to the use of the foot oil. The foot oil is used for protection of the skin against friction. Friction of the skin of the foot can be caused by footwear, for example boots, shoes, pumps, slippers and clogs. But also foot aids, for example a bandage, a plaster bandage, tape, a brace, a splint, an orthosis or a prosthesis can cause friction. The foot oil according to the invention can also be used in combination with these foot aids. The foot oil does not harm the footwear or the foot aids and will not cause stains. The foot oil is effective against friction when the foot is in direct contact with the footwear or the foot aid, but can also be used when wearing socks or a pantyhose.

By the use of the foot oil according to the invention the friction on the skin of the foot is reduced. This is also an advantage when foot and/or toe problems exist. Examples of foot and toe problems are bunions (hallux valgus), plantar fasciitis, heel spur, bursitis in the heel, hammer toes, claw toes and mallet toes. The pain caused by these conditions will decrease when the foot oil according to the invention is used.

The foot oil according to the invention does not dissolve in water and also protects against friction in moist and/or wet conditions.

The foot oil according to the invention does not dry out and is also used as a moisturizer for the skin. Because the foot oil does not dry out the protection against friction remains present for at least 1 hour, preferably for at least 2 hours, more preferably for at least 4 hours. The protection against friction remains for at most 12 hours, preferably at most 18 hours, more preferably for at most 24 hours.

The foot oil according to the invention can be applied on the skin of the foot once a day, but also several times during a day. This depends on the activity of a person during the day. For example, when a person is walking, running or standing for several hours it is likely that the foot oil will be applied more than once. The foot oil can be removed by washing the feet with soap.

The property of the foot oil to protect against friction can not only be used on the feet, but also on other places of the body where clothes touch the skin and cause friction or where skin touches skin and causes friction during exercise or sport activities. The foot oil can, for example, also be used under the armpits, in the neck and between the legs during walking or running, and on the buttocks, for example during cycling.

The invention is further described by the accompanying examples, which are exemplary and explanatory of nature and are not limiting the scope of the invention. To the person skilled in the art it may be clear that many variants, being obvious or not, may be conceivable falling within the scope of protection, defined by the present claims.

### EXAMPLES

### Ingredients

### Dimethicone

D1: dimethicone with a density of 0.915 g/cm³
D2: dimethicone with a density of 0.903 g/cm³

### Mixtures

M1: dimethicone (87 wt%) and dimethiconol (13 wt%) with a density of 0.923 g/cm³
M2: cyclopentasiloxane (85%) en dimethiconol (15%) with a density of 0.963 g/cm³.

### Additive

A mixture of different fragrances and aromatic substances.

### Preparation

### Example 1

30 wt% of D1, 19.9 wt% of D2 and 50wt% of M1 were mixed and 0.1 wt% additives were added. The mixture was blended and the density of the mixture was determined.

### Example 2

43 wt% of D1, 37 wt% of M1 and 20 wt% of M2 were mixed and the additives were added. The mixture was blended and the density of the mixture was determined.

### Example 3

52 wt% of D1, 37wt% of M1 and 4 wt% of M2 were mixed and the additives were added. The mixture was blended and the density of the mixture was determined.

**Table 1**

| **Example** | **Oil** | | | | **Density** |
|---|---|---|---|---|---|
| | Dimethicone (wt%) | Dimethiconol (wt%) | Cyclopentasiloxane (wt%) | Additives (wt%) | (g/cm³) |
| 1 | 93,40 | 6,50 | - | 0.10 | 0.920 |
| 2 | 75.09 | 7.81 | 17.00 | 0.10 | 0.930 |
| 3 | 90.18 | 6.32 | 3.40 | 0.10 | 0.925 |

### Results

The foot oils according to Examples 1-3 were applied on the skin of the feet and between the toes of several test persons. Shoes were put on and were worn for at least 10 hours. The amount of friction experienced during wearing was determined by the test persons wearing the shoes. An average of the friction that was determined by the test persons is given in Table 2.

The friction was determined as follows:
High friction = --
Medium friction = +/-
Low friction = ++

Also the time the low friction feel lasted was monitored by the test persons. The shortest time that was determined is given in Table 2.

The test persons also judged the feeling of the oil during application of the oil on the skin of the feet.

The feeling was determined as follows:
Wet, silky or greasy

**Table 2**

| **Example** | **Properties** | | |
|---|---|---|---|
| | Friction | Time (hours) | Feeling during application |
| 1 | ++ | 10 | Silky |
| 2 | +/- | 6 | Greasy |
| 3 | +/- | 8 | Silky |

## Claims

1. Foot oil consisting of 75.0 - 99.9 wt% dimethicone, 25.0 - 0.1 wt% dimethiconol and optionally 1.10⁻⁶ - 0.1 wt% additives based on the total amount of foot oil.

2. Foot oil according to claim 1, wherein the amount of dimethicone is 80-99 wt%, preferably 85-97 wt%.

3. Foot oil according to any one of the preceding claims, wherein the amount of dimethiconol is 20-1.0 wt%, preferably 15-3.0 wt%.

4. Foot oil according to any one of the preceding claims, wherein the foot oil has a density of 0.920 to 0.940 g/cm³, preferably 0.922 to 0.938 g/cm³.

5. Foot oil according to any one of the preceding claims, wherein the additives are aromatic substances, perfumes and/or combinations thereof.

6. Process for the preparation of a foot oil according to any one of claims 1-6, wherein at least one dimethicone, at least one dimethiconol and optionally the additives are mixed to form the foot oil.

7. Process according to claim 7, wherein at least one dimethicone has a density of 0.895 to 0.925 g/cm³.

8. Process according to claim 7, wherein all dimethicones have a density of 0.895 to 0.925 g/cm³.

9. Use of the foot oil according to any one of claims 1-6, for protection of the skin against friction.

10. Use according to claim 9, wherein the protection against friction remains present for at least 1 hour.

11. Use according to claim 9 or 10, wherein the friction is caused by footwear.

12. Use of the foot oil according to claims 1-6, as a moisturizer.
